# EUROPEAN PATENT SPECIFICATION

(11) **EP 2 429 978 B1**
(45) Date of publication and mention of the grant of the patent: **11.09.2013**
(21) Application number: 10720050.3
(22) Date of filing: 29.04.2010
(51) Int. Cl.: C07C 45/49, C07C 45/50, C07C 49/17, C07F 15/00, B01J 31/24

(54) **HYDROFORMYLATION PROCESS**
HYDROFORMYLIERUNGSVERFAHREN
PROCÉDÉ D'HYDROFORMYLATION

(30) Priority: 13.05.2009 US 454156
(43) Date of publication of application: 21.03.2012
(73) Proprietor: Lyondell Chemical Technology, L.P., Greenville, Delaware 19807 (US)
(72) Inventor: WHITE, Daniel F., West Chester, Pennsylvania 19382 (US)
(74) Representative: Sacco, Marco
(86) International application number: PCT/US2010/001257
(87) International publication number: WO 2010/132087

(56) References cited:
- US-B1- 7 271 295
- US-B1- 7 279 606
- U. HENGARTNER ET AL.: "Synthesis of (R)-6-Methyltryptophan via Enantioselective Catalytic Hydrogenation" THE JOURNAL OF ORGANIC CHEMISTRY, vol. 44, no. 22, 1979, pages 3741-3747, XP002587146 ISSN: 0022-3263

## Description

### FIELD OF THE INVENTION

This invention relates to a process for hydroformylating allyl alcohol to produce 4-hydroxybutyraldehyde.

### BACKGROUND OF THE INVENTION

The hydroformylation of allyl alcohol is a well known and commercially practiced process. See, for example, U.S. Pat. Nos. 4,064,145, 4,215,077, 4,238,419, 4,678,857, and 5,290,743. In the hydroformylation reaction, allyl alcohol is reacted with a CO/H₂ gas mixture in the presence of a catalyst to form 4-hydroxybutyraldehyde (HBA). The HBA may then be separated from the catalyst, e.g., by water extraction, and hydrogenated to form 1,4-butanediol (BDO). See U.S. Pat. No. 5,504,261.

Various catalyst systems have been employed for the hydroformylation reaction, most notably a rhodium complex together with a phosphine ligand (see, e.g., U.S. Pat. Nos. 4,064,145, 4,238,419, and 4,567,305). Commonly employed phosphine ligands are trisubstituted phosphines such as triphenyl phosphine.

One disadvantage of the hydroformylation process is that other co-products or byproducts are also formed in addition to the desired HBA linear product. The hydroformylation of allyl alcohol typically produces some 3-hydroxy-2-methylpropionaldehyde (HMPA) branched co-product and C₃ byproducts such as n-propanol and propionaldehyde. Although HMPA may be hydrogenated to produce 2-methyl-1,3-propanediol (MPD), which is a useful material, the MPD co-product reduces the yield of BDO. Formation of the C₃ byproducts effectively represents another yield loss in the process which can have a severe adverse effect on the process economics.

To increase BDO yields, research continues to improve the hydroformylation process and reduce less desired co-product/byproducts. U.S. Pat. No. 6,127,584 discloses that the use of a trialkyl phosphine ligand having at least 2 methyl groups results in increased HBA:HMPA ratio. The use of diphosphine ligands has also been found to improve the HBA:HMPA ratio. The hydroformylation of allyl alcohol using rhodium complex catalysts and diphosphine ligands such as DIOP, XANTPHOS, or trans-1,2-bis(diphenylphosphinomethyl)cyclobutane is shown in the art, notably in Japan Kokai Nos. 06-279345 and 06-279344 and U.S. Pat. No. 4,306,087. U.S. Pat. No. 6,225,509 discloses that maintaining the concentration of CO in the reaction liquid above about 4.5 mmols/liter reduces the make of undesirable C₃ co-products when using a catalyst comprised of a rhodium complex and a ligand such as DIOP. In addition, U.S. Pat. Nos. 7,271,295 and 7,279,606 disclose the use of a 2,3-*O*-isopropylidene-2,3-dihydroxy-1,4-bis[bis(3,5-di-n-alkylphenyl)phosphino]butane ligand or a trans-1,2-bis(bis(3,5-di-n-alkylphenyl)phosphinomethyl)cyclobutane ligand, respectively.

In sum, new processes for hydroformylating allyl alcohol to produce 4-hydroxybutyraldehyde are needed. Particularly valuable processes would result in high ratios of 4-hydroxybutyraldehyde to 3-hydroxy-2-methylpropionaldehyde.

### SUMMARY OF THE INVENTION

The invention is a process that comprises reacting allyl alcohol with carbon monoxide and hydrogen in the presence of a solvent and a catalyst to produce 4-hydroxybutyraldehyde. The catalyst comprises a rhodium complex and a trans-1,2-bis(bis(3,4-di-n-alkylphenyl)phosphinomethyl)cyclobutane and/or a 2,3-*O-*isopropylidene-2,3-dihydroxy-1,4-bis[bis(3,4-di-n-alkylphenyl)phosphino]butane. The invention also includes the catalyst according to claims 11-14. The invention surprisingly results in high ratios of 4-hydroxybutyraldehyde product to 3-hydroxy-2-methylpropionaldehyde.

### DETAILED DESCRIPTION OF THE INVENTION

The process of the invention comprises hydroformylating allyl alcohol in the presence of a solvent and a catalyst. The catalyst of the invention according to claims 11-14 comprises a rhodium complex and a diphosphine ligand. The diphosphine is a trans-1,2-bis(bis(3,4-di-n-alkylphenyl)phosphinomethyl)cyclobutane and/or a 2,3-*O-*isopropylidene-2,3-dihydroxy-1,4-bis[bis(3,4-di-n-alkylphenyl)phosphino]butane.

Trans-1,2-bis(bis(3,4-di-n-alkylphenyl)phosphinomethyl) cyclobutanes have the formula: 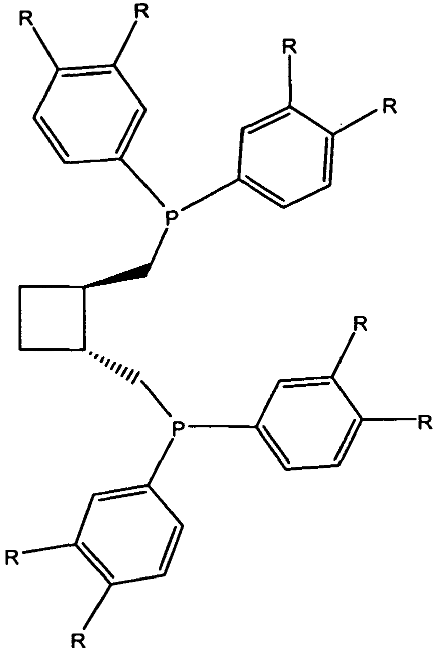 wherein each R is independently an n-alkyl group. Preferably, R is methyl, ethyl, or propyl.

The trans-1,2-bis(bis(3,4-di-n-alkylphenyl)phosphinomethyl) cyclobutane diphosphine ligand is most preferably trans-1,2-bis(bis(3,4-dimethylphenyl)phosphinomethyl)cyclobutane or trans-1,2-bis(bis(3,4-diethylphenyl)phosphinomethyl)cyclobutane.

2,3-O-isopropylidene-2,3-dihydroxy-1,4-bis[bis(3,4-di-n-alkylphenyl) phosphino]butanes have the formula: 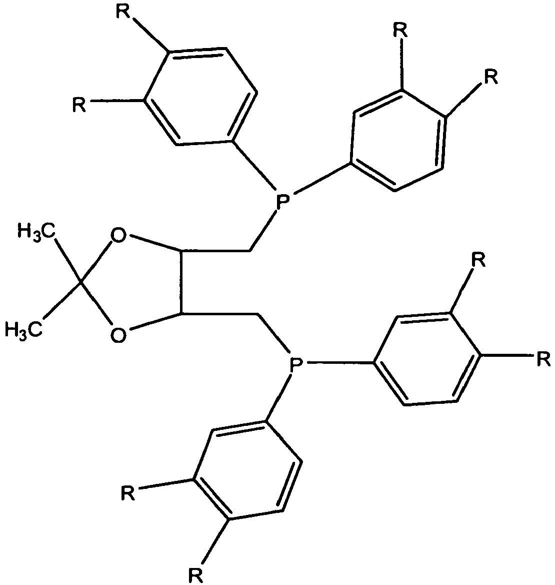 wherein each R is independently an n-alkyl group. Preferably, R is methyl, ethyl, or propyl.

The 2,3-*O*-isopropylidene-2,3-dihydroxy-1,4-bis[bis(3,4-di-n-alkylphenyl) phosphino]butane diphosphine ligand is most preferably 2,3-*O*-isopropylidene-2,3-dihydroxy-1,4-bis[bis(3,4-dimethylphenyl) phosphino]butane or 2,3-*O*-isopropylidene-2,3-dihydroxy-1,4-bis[bis(3,4-diethylphenyl) phosphino]butane.

The diphosphine ligand may be prepared by any possible method. For instance, 2,3-*O*-isopropylidene-2,3-dihydroxy-1,4-bis[bis(3,4-di-n-alkylphenyl) phosphino]butanes may be prepared by the reaction of 2,2-dimethyl-4,5-bis[(toluenesulfonyloxymethyl) methyl]-1,3-dioxolane with lithium di(3,4-di-n-alkylphenyl)phosphines. Trans-1,2-bis(bis(3,4-di-n-alkylphenyl)phosphinomethyl) cyclobutanes may be prepared by the reaction of trans-1,2-cyclobutanedimethanol, bis(toluenesulfonate) with lithium di(3,4-di-n-alkylphenyl)phosphines.

The catalyst of the invention also comprises a rhodium complex. Suitable rhodium complexes contain rhodium attached to ligand groups. The rhodium complex is preferably soluble in the solvent. There are no particular restrictions regarding the choice of ligands attached to the rhodium complex. For example, suitable ligands include hydrides, carbonyl, substituted and unsubstituted cyclopentadienyls, 2,4-alkanedionates, trialkyl phosphines, triaryl phosphines, diphosphines, and mixtures thereof. Particularly preferred ligands include carbonyl, acetylacetonate (2,4-pentanedionate), triphenylphosphine, and mixtures thereof. Examples of preferred rhodium complexes include (acetylacetonato) dicarbonylrhodium and tris(triphenylphosphine)rhodium carbonyl hydride.

The rhodium complex can be pre-associated with the trans-1,2-bis(bis(3,4-din-alkylphenyl)phosphinomethyl)cyclobutane or 2,3-*O*-isopropylidene-2,3-dihydroxy-1,4-bis[bis(3,4-di-n-alkylphenyl)phosphino] butane diphosphine ligand prior to use in the hydroformylation reaction such that the bis(bis(3,4-di-n-alkylphenyl)phosphinomethyl)cyclobutane or 2,3-*O*-isopropylidene-2,3-dihydroxy-1,4-bis[bis(3,4-di-n-alkylphenyl)phosphino] butane forms part of the rhodium complex, or it can be added separately. However, it is preferable to add the rhodium complex separate from the trans-1,2-bis(bis(3,4-di-n-alkylphenyl)phosphinomethyl)cyclobutane or 2,3-*O*-isopropylidene-2,3-dihydroxy-1,4-bis[bis(3,4-di-n-alkylphenyl)phosphino]butane. The molar ratio of the diphosphine ligand:rhodium complex is preferably in the range of 0.5:1 to 5:1.

Although not necessary, the catalyst may additionally comprise a monophosphine compound. The monophosphine compound is in addition to any phosphine ligand that may be associated with the rhodium complex. The monophosphine compound is a trisubstituted phosphine that is represented by the formula:

(R¹)₃P

wherein R¹ is an aryl or alkyl group. Suitable aliphatic R¹ groups include methyl, ethyl, n-butyl, sec-butyl, octyl, and decyl. Suitable aromatic R¹ groups include phenyl, tolyl, and naphthyl. The R¹ groups may be the same or are different, but preferably are the same. Preferably, the monophosphine is a trisubstituted aryl phosphine. More preferably, the monophosphine is triphenylphosphine or tritolylphosphine. Triphenyl phosphine is particularly preferred.

A reaction solvent is also required for the process of the invention. Typical solvents are those that are capable of solubilizing the rhodium complex and are not reactive to the hydroxyaldehydes that are produced in the hydroformylation step. Suitable solvents include any organic solvent having very low or minimal solubility in water. Preferred solvents include C₅-C₂₀ aliphatic hydrocarbons, C₆-C₂₀ aromatic hydrocarbons, alcohols, ethers, and mixtures thereof. Particularly preferred solvents include toluene, cyclohexane, methyl t-butyl ether, and mixtures thereof.

Typical reaction conditions for the hydroformylation step are mild to favor the formation of the linear 4-hydroxybutyraldehyde (HBA) rather than branched 3-hydroxy-2-methylpropionaldehyde (HMPA) reaction product. Reaction conditions are preferably in the range of from 20 to 120°C and pressures of from 138 to 4137 kPa [20 to 600 psig], more preferably from 45 to 85°C and 207 to 2758 kPa [30 to 400 psig], and most preferably from 50 to 80°C and 276 to 2068 kPa [40 to 300 psig]. The molar ratio of CO:H₂ is typically about 1:1, although the ratio can vary considerably. The partial pressure of CO is typically within the range of 34 to 690 kPa [5 to 100 psig]. The partial pressure of hydrogen is typically within the range of 276 to 1379 kPa [40 to 200 psig]. The reaction is conducted at these conditions until a predominance of the allyl alcohol has reacted, e.g. 60 to 99.9%, the products being largely 4-hydroxybutyraldehyde with some branched reaction products. The amount of reaction time is not critical, but usually a reaction time of 0.5 to 4 hours is adequate.

Preferably, the allyl alcohol starting concentration on a reaction solvent to feed basis is in the range of 5 to 40 percent by weight in the solvent; more preferably, lower concentration in the range of 5 to 10 percent by weight may be used.

Preferably, the hydroformylation of allyl alcohol is carried out such that the concentration of CO in the liquid phase ([CO]_{liq}) is maintained above 4 mmols/liter (0.004 M) during the hydroformylation. The value of [CO]_{liq} is defined in U.S. Pat. No. 6,225,509. Preferably, the liquid phase hydrogen:carbon monoxide molar ratio is in the range of from 10:1 to 1:2, more preferably from 5:1 to 1:2.

Following the hydroformylation step, the HBA product is preferably separated from the solvent and catalyst by water extraction in an extraction vessel. Water extraction methods are well known in the art and can be affected by any suitable means, such as mixer-settlers, packed or trayed extraction columns, rotating disk contactors, or passed to a settling tank for resolution of the mixture into aqueous and organic phases. HBA, and any HMPA, remains soluble in the water (aqueous) phase and is separated from the solvent (organic) phase.

The 4-hydroxybutyraldehyde (and any 3-hydroxy-2-methylpropionaldehyde) reaction product is preferably subjected to an additional step of hydrogenating the 4-hydroxybutyraldehyde in the presence of a hydrogenation catalyst to produce 1,4-butanediol (BDO). Hydrogen is added to the reaction vessel for the hydrogenation. Suitable hydrogenation catalysts include any Group VIII metal, such as nickel, cobalt, ruthenium, platinum, and palladium, as well as copper, zinc and chromium and mixtures and alloys thereof. Especially preferred are nickel catalysts. Most preferred are Raney® nickel-type and fixed bed nickel catalysts.

The hydrogenation reaction conditions are preferably in the range of from 60 to 200°C and pressures of from 1379 to 6895 kPa [200 to 1000 psig], more preferably from 80 to 140°C and 2068 to 6895 kPa [300 to 1000 psig]. Generally reaction times of 1 to 10 hours are appropriate. During the hydrogenation reaction, BDO and MPD formed while the high ratio of linear to branched products is substantially retained, along with other low co-product/byproducts.

### EXAMPLE 1: PREPARATION OF DIPHOSPHINES

1A, 1B, 1C, 1D, and 1E: Diphosphines 1A, 1B, 1C, 1D, and 1E of the following general formula are prepared as described below.

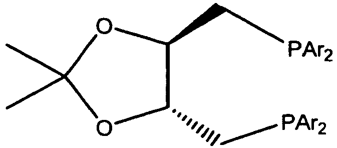

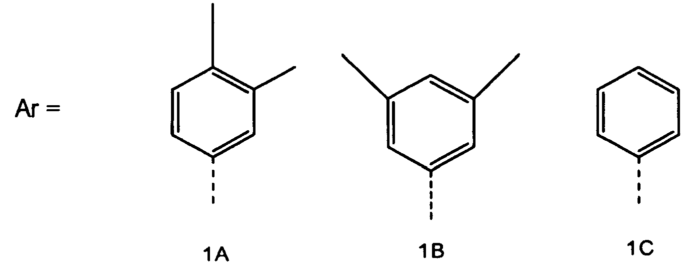

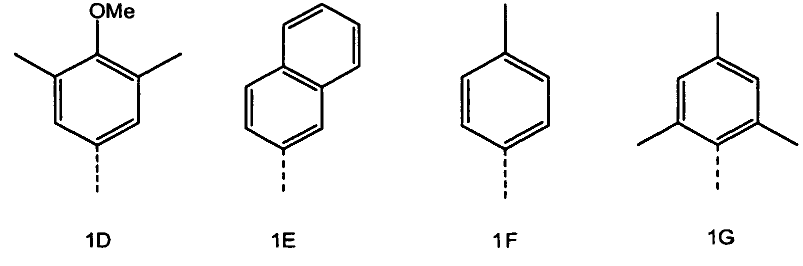

A solution of 2,2-dimethyl-4,5-bis[(toluenesulfonyloxymethyl) methyl]-1,3-dioxolane in dry/degassed THF (1 equivalent, 1.73 g, 3.7 x 10⁻³ moles of the dioxolane in 50 mL THF) is added drop-wise under argon to a solution of the appropriate lithium diarylphosphine (see formula above) in dry/ degassed THF (2.3 equivalents in 100 mL THF). The mixture is heated at reflux for 2 hours, then cooled, and the solvent is removed under reduced pressure. The remaining solids are re-dissolved in dichloromethane, filtered though a silica bed, and the solvent is removed under reduced pressure to yield the 2,3-*O*-isopropylidene-2,3-dihydroxy-1,4-bis(diarylphosphino)butane.

Diphosphine 1A: 2,3-*O*-isopropylidene-2,3-dihydroxy-1,4-bis[bis(3,4-dimethylphenyl)phosphino]butane.

Comparative Diphosphine 1B: 2,3-*O*-isopropylidene-2,3-dihydroxy-1,4-bis(bis(3,5-dimethylphenyl)phosphino]butane.

Comparative Diphosphine 1C: 2,3-*O*-isopropylidene-2,3-dihydroxy-1,4-bis[bis(phenyl)phosphino], known as DIOP.

Comparative Diphosphine 1D: 2,3-*O*-isopropylidene-2,3-dihydroxy-1,4-bis[bis(3,5-dimethyl-4-methoxyphenyl)phosphino]butane.

Comparative Diphosphine 1E: 2,3-*O*-isopropylidene-2,3-dihydroxy-1,4-bis[bis(2-napthenyl)phosphinojbutane.

Comparative Diphosphine 1F: 2,3-*O*-isopropylidene-2,3-dihydroxy-1,4-bis[bis(4-methylphenyl)phosphino]butane.

Comparative Diphosohine 1G: 2,3-*O*-isopropylidene-2,3-dihydroxy-1,4-bis[bis(2,4,6-trimethylphenyl)phosphino]butane.

### EXAMPLE 2: Hydroformylation Reaction using Diphosphines

Allyl alcohol is hydroformylated using diphosphines 1A-1E according to the following procedure:

A solution of the desired diphosphine (2 equivalents or 8.6 x 10⁻⁵ moles) in dry degassed toluene (15 g) is added to [Rh(CO)₂(acac)] (1 equivalent or 4.3 x 10⁻⁵ moles) in a 100 mL Parr autoclave. The solution is flushed three times with a 1:1 CO/H₂ mixture and then pressurized to 1241 kPa [180 psig] with the CO/H₂ mixture. The autoclave is then heated to 65°C with stirring, allyl alcohol (3.5 mL) is injected, and the autoclave is pressurized to 1379 kPa (200 psig) with the CO/H₂ mixture. The autoclave is kept at a constant pressure of 1379 kPa (200 psig) and the gas uptake of the reaction is monitored. When there is no further gas uptake, the autoclave is cooled and depressurized. The resulting solution is analyzed by gas chromatography to determine the products of the reaction. The reaction produces HBA, HMPA, and C₃ products (n-propanol and propionaldehyde).

**TABLE 1: Diphosphine Comparisons**

| **Diphosphine** | **Conversion (%)** | **HBA (%)** | **HMPA (%)** | **C₃ (%)** | **HBA:HMPA ratio** |
|---|---|---|---|---|---|
| 1A | 99.8 | 89.7 | 9.5 | 0.2 | 9.5 |
| 1B * | 99.7 | 89.8 | 9.4 | 0.2 | 9.5 |
| 1C * | 99.8 | 86.2 | 11.7 | 0.2 | 7.4 |
| 1D * | 99.9 | 82.2 | 14.7 | 0.3 | 5.6 |
| 1E * | 99.8 | 86.0 | 13.2 | 0.3 | 6.5 |
| 1F * | 99.98 | 86.51 | 11.27 | 0.19 | 7.7 |
| 1G * | 6.33 | 2.15 | 0.53 | 3.43 | 4.1 |

| | | | | | |
|---|---|---|---|---|---|
| * Comparative Example | | | | | |

## Claims

1. A process to produce 4-hydroxybutyraldehyde comprising reacting allyl alcohol with carbon monoxide and hydrogen in the presence of a solvent and a catalyst comprising a rhodium complex and a diphosphine selected from the group consisting of a trans-1,2-bis(bis(3,4-di-n-alkylphenyl)phosphinomethyl)cyclobutane, a 2,3-*O*-isopropylidene-2,3-dihydroxy-1,4-bis[bis(3,4-di-n-alkylphenyl)phosphino]butane, and mixtures thereof.

2. The process of claim **1** wherein the diphoshine is trans-1,2-bis(bis(3,4-di-methylphenyl)phosphinomethyl)cyclobutane.

3. The process of claim **1** wherein the diphoshine is trans-1,2-bis(bis(3,4-di-ethylphenyl)phosphinomethyl)cyclobutane.

4. The process of claim **1** wherein the diphoshine is 2,3-*O-*isopropylidene-2,3-dihydroxy-1,4-bis[bis(3,4-dimethylphenyl)phosphino] butane.

5. The process of claim **1** wherein the diphoshine is 2,3-*O-*isopropylidene-2,3-dihydroxy-1,4-bis[bis(3,4-diethylphenyl)phosphino] butane.

6. The process of claim **1** wherein the solvent is selected from the group consisting of C₅-C₂₀ aliphatic hydrocarbons, C₆-C₁₂ aromatic hydrocarbons, ethers, alcohols, and mixtures thereof.

7. The process of claim **1** wherein the solvent is selected from the group consisting of toluene, cyclohexane, methyl t-butyl ether, and mixtures thereof.

8. The process of claim **1** wherein the rhodium complex comprises rhodium and ligands selected from the group consisting of hydride, carbonyl, trialkyl phosphines, triaryl phosphines, diphosphines, cyclopentadienyls, 2,4-alkanedionates, and mixtures thereof.

9. The process of claim **1** wherein the catalyst further comprises a monophosphine compound.

10. The process of claim **1** further comprising hydrogenating the 4-hydroxybutyraldehyde in the presence of a hydrogenation catalyst to form 1,4-butanediol.

11. A catalyst comprising a rhodium complex and a trans-1,2-bis(bis(3,4-di-n-alkylphenyl)phosphinomethyl)cyclobutane.

12. The catalyst of claim **11** which comprises the rhodium complex and trans-1,2-bis(bis(3,4-di-methylphenyl)phosphinomethyl)cyclobutane.

13. The catalyst of claim **11** which comprises the rhodium complex and trans-1,2-bis(bis(3,4-di-ethylphenyl)phosphinomethyl)cyclobutane.

14. A catalyst comprising a rhodium complex and 2,3-*O-*isopropylidene-2,3-dihydroxy-1,4-bis[bis(3,4-diethylphenyl)phosphino]butane.

## Patentansprüche

1. Verfahren zur Herstellung von 4-Hydroxybutyraldehyd, umfassend das zur Reaktion bringen von Allylalkohol mit Kohlenmonoxid und Wasserstoff bei Vorliegen eines Lösungsmittels und eines Katalysators, umfassend einen Rhodiumkomplex und ein Diphosphin, das aus der Gruppe ausgewählt ist, bestehend aus einem Trans-1,2-bis(bis(3,4-di-n-alkylphenyl)phosphinomethyl)cyclobutan, einem 2,3-O-Isopropyliden-2,3-dihydroxy-1,4-bis[bis(3,4-di-n-alkylphenyl)phosphino]butan und Gemischen davon.

2. Verfahren nach Anspruch 1, wobei das Diphosphin Trans-1,2-bis(bis(3,4-di-methylphenyl)phosphinomethyl)-cyclobutan ist.

3. Verfahren nach Anspruch 1, wobei das Diphosphin Trans-1,2-bis(bis(3,4-di-ethylphenyl)phosphinomethyl)-cyclobutan ist.

4. Verfahren nach Anspruch 1, wobei das Diphosphin 2,3-O-Isopropyliden-2,3-dihydroxy-1,4-bis[bis(3,4-dimethylphenyl)phosphino]butan ist.

5. Verfahren nach Anspruch 1, wobei das Diphosphin 2,3-O-Isopropyliden-2,3-dihydroxy-1,4-bis[bis(3,4-diethylphenyl)phosphino]butan ist.

6. Verfahren nach Anspruch 1, wobei das Lösungsmittel aus der Gruppe ausgewählt ist, bestehend aus aliphatischen C₅- bis C₂₀-Kohlenwasserstoffen, aromatischen C₆- bis C₁₂-Kohlenwasserstoffen, Ethern, Alkoholen und Gemischen davon.

7. Verfahren nach Anspruch 1, wobei das Lösungsmittel aus der Gruppe ausgewählt ist, bestehend aus Toluen, Cyclohexan, Methyl-t-butylether und Gemischen davon.

8. Verfahren nach Anspruch 1, wobei der Rhodiumkomplex Rhodium und Liganden umfasst, die aus der Gruppe ausgewählt sind, bestehend aus Hydrid, Carbonyl, Trialkylphosphinen, Triarylphosphinen, Diphosphinen, Cyclopentadienylen, 2,4-Alkandionaten und Gemischen davon.

9. Verfahren nach Anspruch 1, wobei der Katalysator ferner eine Monophosphinverbindung umfasst.

10. Verfahren nach Anspruch 1, ferner umfassend die Hydrierung des 4-Hydroxybutyraldehyds bei Vorliegen eines Hydrierkatalysators zur Bildung von 1,4-Butandiol.

11. Katalysator, der einen Rhodiumkomplex und ein Trans-1,2-bis(bis(3,4-di-n-alkylphenyl)phosphinomethyl)-cyclobutan umfasst.

12. Katalysator nach Anspruch 11, der den Rhodiumkomplex und Trans-1,2-bis(bis(3,4-di-methylphenyl)phosphinomethyl)cyclobutan umfasst.

13. Katalysator nach Anspruch 11, der den Rhodiumkomplex und Trans-1,2-bis(bis(3,4-di-ethylphenyl)-phosphinomethyl)cyclobutan umfasst.

14. Katalysator, der einen Rhodiumkomplex und 2,3-*O-*Isopropyliden-2,3-dihydroxy-1,4-bis[bis(3,4-diethylphenyl)phosphino]butan umfasst.

## Revendications

1. Procédé de production de 4-hydroxybutyraldéhyde comprenant la réaction d'alcool allylique avec du monoxyde de carbone et de l'hydrogène en présence d'un solvant et d'un catalyseur comprenant un complexe de rhodium et une diphosphine choisie dans le groupe constitué par le trans-1,2-bis(bis(3,4-di-n-alkylphényl)phosphinométhyl)cyclobutane, le 2,3-O-isopropylidène-2,3-dihydroxy-1,4-bis[bis(3,4-di-n-alkylphényl)phosphino]butane, et leurs mélanges.

2. Procédé selon la revendication 1, la diphosphine étant le trans-1,2-bis(bis(3,4-diméthylphényl)phosphinométhyl)cyclobutane.

3. Procédé selon la revendication 1, la diphosphine étant le trans-1,2-bis(bis(3,4-diéthylphényl)phosphinométhyl)cyclobutane.

4. Procédé selon la revendication 1, la diphosphine étant le 2,3-O-isopropylidène-2,3-dihydroxy-1,4-bis[bis(3,4-diméthylphényl)phosphino]butane.

5. Procédé selon la revendication 1, la diphosphine étant le 2,3-O-isopropylidène-2,3-dihydroxy-1,4-bis[bis(3,4-diéthylphényl)phosphino]butane.

6. Procédé selon la revendication 1, le solvant étant choisi dans le groupe constitué par les hydrocarbures aliphatiques en C₅-C₂₀, les hydrocarbures aromatiques en C₆-C₁₂, les éthers, les alcools et leurs mélanges.

7. Procédé selon la revendication 1, le solvant étant choisi dans le groupe constitué par le toluène, le cyclohexane, le méthyl-t-butyléther et leurs mélanges.

8. Procédé selon la revendication 1, le complexe de rhodium comprenant du rhodium et des ligands choisis dans le groupe constitué par hydrure, carbonyle, trialkylphosphines, triarylphosphines, diphosphines, cyclopentadiényles, 2,4-alcanedionates et leurs mélanges.

9. Procédé selon la revendication 1, le catalyseur comprenant en outre un composé de type monophosphine.

10. Procédé selon la revendication 1, comprenant en outre l'hydrogénation du 4-hydroxybutyraldéhyde en présence d'un catalyseur d'hydrogénation pour former le 1,4-butanediol.

11. Catalyseur comprenant un complexe de rhodium et le trans-1,2-bis(bis(3,4-di-n-alkylphényl)phosphinométhyl)cyclobutane.

12. Catalyseur selon la revendication 11, comprenant le complexe de rhodium et le trans-1,2-bis(bis(3,4-diméthylphényl)phosphinométhyl)cyclobutane.

13. Catalyseur selon la revendication 11, comprenant le complexe de rhodium et le trans-1,2-bis(bis(3,4-diéthylphényl)phosphinométhyl)cyclobutane.

14. Catalyseur comprenant un complexe de rhodium et le 2,3-O-isopropylidène-2,3-dihydroxy-1,4-bis[bis(3,4-diéthylphényl)phosphino]butane.
